Europäisches Patentamt

European Patent Office

Office européen des brevets

(19)

(11) Publication number: **0 079 671**
**A2**

(12) **EUROPEAN PATENT APPLICATION**

(21) Application number: 82305096.8

(22) Date of filing: 27.09.82

(51) Int. Cl.³: **C 07 C 102/00**
**C 07 C 103/44**

(30) Priority: 28.09.81 GB 8129271

(43) Date of publication of application:
25.05.83 Bulletin 83/21

(84) Designated Contracting States:
DE FR GB IT

(71) Applicant: Albright & Wilson Limited
Albright & Wilson House Hagley Road West
Oldbury Warley West Midlands, B68 ONN(GB)

(72) Inventor: Philips, Brinley Morris
12 Eden Drive Moresby Parks
Whitehaven Cumbria(GB)

(72) Inventor: Crosby, Michael James
1 Thornton Road
Fairfield Whitehaven(GB)

(72) Inventor: Hoye, Peter Albert Theodore
Tresco Orchard Grove Dunsley Kinver
Stourbridge West Midlands(GB)

(74) Representative: Wilson, Michael John et al,
c/o Albright & Wilson Limited 1 Knightsbridge Green
London SW1X 7QD(GB)

(54) Process for preparing tetraacetyl alkylene diamines.

(57) Tetracetylethylene diamine, an additive for detergent bleaching compositions is made from the di acetyl compound by heating with acetic anhydride, removing acetic acid byproduct as distillate with acetic anhydride and adding fresh acetic anhydride, preferably recovered from the distillate.

EP 0 079 671 A2

This invention relates to a process for preparing an amide, in particular tetra acetyl alkylene diamine.

Tetra acetylalkylene diamines, especially tetra acetylethylene diamine, are known as additives for washing compositions containing an oxygen yielding bleaching agent. Tetra acetyl ethylene diamine may be made by reaction of diacetyl ethylene diamine and acetic anhydride for several hours under boiling conditions (see Rec. Trav. Chim. 1911, 30, pages 183-185). Prolonged heating causes substantial discolouration of the product in reduced yield. In BP 1357595, diacetyl ethylene diamine is reacted with acetic anhydride in a vessel with reflux condenser under total reflux, and the acetic acid vapour produced is reacted in the condenser with ketene to reform acetic anhydride which returns to the vessel; ketene can only conveniently be made and used on a large scale. In BP 1335204 the diacetylethylene diamine and acetic anhydride are heated together at 140°C with distillation of the by product acetic acid, followed by cooling, separation of crystals of tetra acetyl ethylene diamine, and evaporation of the mother liquor, further cooling and separation of more crystals in a total yield of 91%. In BP 1378303 tetra acetyl ethylene diamine is prepared in a multicycle process in about 85% overall yield by heating diacetyl ethylene diamine and acetic anhydride with distillation of acetic acid, followed by cooling, separation of the crystals of product from mother liquor, recycle of 50-90% of the mother liquor, to which replenishment amounts of diacetyl ethylene diamine and acetic anhydride are added and the reaction cycle repeated.

We have found that this latter process gives a significant amount of byproduct triacetyl ethylene diamine and its degradation products, 10-50% of which is removed in the separated portions of the mother liquor which is not recycled. Because in the separated mother liquor the tri compound is contaminated with coloured products, the tri compound cannot cheaply be recovered and therefore represents lost product.

We have now found a very much simpler process which gives very high yields without use of ketene and without the recycle of these contaminated mother liquors. The reactants are heated together with evaporation of acetic acid and acetic anhydride and addition of fresh acetic anhydride to the reactants and maintenance of a particular molar excess of acetic anhydride.

The present invention provides a process for preparing a tetra acetyl alkylene diamine with 1-6 carbon atoms in the alkylene group, which process comprises heating a mixture comprising an amide which is a diacetylalkylene diamine, with acetic anhydride, preferably in a molar ratio of 1 : 3 to 1 : 10, to form a reaction mixture comprising at least one amide and acetic acid, while evaporating a vapour comprising acetic acid and acetic anhydride, and adding further acetic anhydride to the reaction mixture during the reaction and maintaining the molar ratio of acetic anhydride to the total of amides containing said alkylene groups at 3 : 1 to 10 : 1 during the reaction, and then recovering tetra acetyl alkylene diamine from the reaction mixture at the end of the reaction. In the recovery the reaction mixture after concentration if necessary or desired is usually cooled and allowed to crystallize. Crystals of tetra acetyl alkylene diamine are separated.

While the molar proportion of acetic anhydride to diacetyl alkylene diamine at the start of the reaction may be 2-3 : 1, preferably it is 3 : 1 to 10 : 1 such as 3 : 1 to 7 : 1 or 3.5 : 1 to 6 : 1, 3 : 1 to 5.9 : 1 or especially 3 : 1 to 4.5 : 1. Advantageously the mixture at the start also comprises acetic acid, usually in an amount of 0.5-10% e.g. 0.5-5% by weight based on the total weight of the acetic anhydride and acetic acid.

The diacetyl alkylene diamine with 1-6 carbon atoms in the alkylene group usually has the nitrogen atoms attached to different carbon atoms of the alkylene chain; those nitrogen atoms are usually at the end of the alkylene chain. Examples are the acetyl derivatives of methylene diamine 1, 2-ethylene diamine, 1, 3-propylene diamine and 1, 6-hexylene diamine. Usually the two acetyl groups are on different nitrogen atoms. NN'-diacetyl ethylene diamine is preferred. The diacetyl alkylene diamines in which the alkylene group has 2-6 carbon atoms and in which the nitrogen atoms are attached to different carbon atoms in the alkylene group may be made by acetylation of the alkylene diamine e.g. with acetic acid or acetic anhydride with heating at 100-180°C and preferably subsequently with evaporation of water, acetic acid and other volatiles e.g. to give a molten amide. Alternatively after evaporation of the water, and usually some of the acetic acid, the solution of diamide in acetic acid may be used as such in the reaction with acetic anhydride. The diacetyl alkylene diamines, in which the nitrogen atoms are attached to the same carbon atom, may be made by reacting acetamide with an aldehyde such as formaldehyde or ketone of the same number of carbon atoms as the alkylene group in the amide product.

The diacetyl alkylene diamine may be mixed with the acetic anhydride in any convenient manner, but preferably the former is in a solid or molten form and is in the substantial absence of solvent apart from the possibility of some acetic acid e.g. in an amount as mentioned above. Thus the acetic anhydride may be mixed with powdered, or less preferably lumps, of diacetyl ethylene diamine or with the compound molten as a pool or as drops or in solution in acetic acid. The diamide and acetic anhydride may be mixed in any order, including simultaneously.

The mixture of acetic anhydride and diacetyl alkylene diamine are heated together up to 100-180°C, e.g. 120-160°C especially 140-160°C. The reaction produces acetic acid and this together with acetic anhydride, is evaporated; while the evaporation may be under reduced pressure, operation under a pressure of at least atmospheric pressure is preferred. The condensation system into which the vapours pass is usually such that at least 70% of the vapours are removed from the reaction vessel i.e. a reflux ratio of at most 3 : 7 is used. Preferably the vapour mixture of acetic anhydride and acetic acid, which is removed from the vessel via the condensation system, is fractionated into a lower boiling fraction comprising acetic acid e.g. in a high percentage and a higher boiling fraction comprising acetic anhydride e.g. with 0.5-10% or 1-7% by weight acetic acid; conveniently the higher boiling fraction has an analysis of about 5% acetic acid and 95% acetic anhydride. The lower boiling fraction rich in acetic acid is preferably recycled to react with the alkylene diamine to make the diacetyl alkylene diamine. In this partial separation of acetic acid and acetic anhydride, the vapour or condensate thereof to be fractionated is passed into a separation column, at a place distant from its top and bottom ends and said lower and higher boiling fractions are collected from said top and bottom ends of said column.

Preferably the rate of removal of acetic acid from the reaction mixture is such that during at least 70% of the reaction, the rate of removal is greater than the rate of production of acetic acid. During the reaction, the amount of acetic acid which is preferably present in the reaction mixture during all the reaction can be less than 15% e.g. 0.1-15% by weight and preferably decreases after 30% of reaction has occurred and after 80% of reaction the percentage of acetic acid is less than 5%.

In the process of the present invention the acetic acid is removed preferably as fast as possible. Preferably the rate of removal of acetic acid is faster than the rate of production of acetic acid for the time corresponding to at least 70% of the conversion to the tetraacetylalkylenediamine, and preferably for at least 70% of the total reaction time e.g. for the period of the reaction from 4.5 hrs., particularly 3 hrs. and especially from 2 hrs. from the start of the reaction. During these periods of time at least, the concentration of acetic acid in the reaction mixture is diminishing.

Thus the acetic acid concentration may be less than 15% after 70% conversion, less than 10% after 80% conversion, less than 5% after 90% conversion, and after 95% conversion less than 2.5%, the acetic acid concentration being given as a weight % based on the weight of acetic acid and anhydride. By removing the acetic acid quickly, at least 80% and preferably at least 90% of the reaction has occurred by 6 hrs., usually by 8 hrs. It is preferred that in the function [tetraacetylalkylenediamine] [acetic acid] < x [triacetylalkylene diamine] [acetic anhydride] where the concentrations of the various species in the reaction mixture at any time in the reaction are expressed as molar percentages in the reaction mixture , then x is less than 1, e.g. 0.1-1, preferably 0.3-0.8 such as about 0.5. The acetic acid is removed quickly with acetic anhydride in the vapour phase while maintaining the excess of acetic anhydride as specified below. Fresh acetic anhydride is usually added after at least 50% of reaction preferably after at least 30% reaction and advantageously throughout substantially all the reaction to maintain the desired excess (as needed) and if required at least partly to compensate for the reduction in volume of the reaction mixture. More acetic anhydride is coremoved than would be the case if a slow distillation of acetic acid were involved. Preferably the rate of removal of vapour e.g. distillate is such as to evaporate an amount of vapour distillate 2-20% e.g. 4-15% of the weight of acetic anhydride in the reaction mixture per hour, with addition of 2-20% e.g. 4-15% of that weight of fresh acetic anhydride, while maintaining the excess of acetic anhydride and usually keeping the volume of reaction liquid constant $\pm$ 10%. By this means over the total reaction at least 0.2, e.g. 0.2-3 such as 0.4-2.0 or at least 0.45 such as 0.7-1.5 unit weights of fresh acetic anhydride may be added for each unit weight of acetic anhydride originally mixed with the diacetylalkylenediamine.

During the reaction, acetic anhydride is used up and evaporated, acetic acid is produced and evaporated and fresh acetic anhydride is added. The amount of acetic anhydride at any time in the reaction mixture is preferably maintained in the 3 : 1 to 5.9 :

l e.g. 3 : 1 to 5.5 : 1 or 3 : 1 to 5 : 1 ratio most preferably 3.0-
4.5 : 1 or 3.4 : 1 to 5.5 : 1 to the total of amides containing
said alkylene group (i.e. di, tri and tetra amide derivatives).
The fresh acetic anhydride may be added continually, in discrete
portions or continuously. Advantageously at least 50% by weight and
preferably at least 90% e.g. substantially all the higher boiling
fraction collected from the reaction vapours and comprising acetic
anhydride and 0.5-10% preferably 5% acetic acid is recycled and
passed into the reaction mixture as the source of the further acetic
anhydride. Extra acetic anhydride to compensate for the acetic acid
separated in the lower boiling fraction may conveniently be mixed
with the higher boiling fraction comprising acetic anhydride to form
a stream (containing e.g. 0.5-5% acetic acid by weight) which is
recycled into the reaction mixture. The volume of the liquid in the
reaction vessel may preferably be maintained to a constant value $\pm$
10% during the reaction. Advantageously the liquid recycled into
the reaction mixture contains 0.5-5% acetic acid.

Thus in an integrated process the acetic anhydride and diacetyl
alkylene diamine are heated together in a reaction vessel to give
a vapour of acetic acid and acetic anhydride which is fractionated
to give a lower boiling fraction comprising acetic acid and a higher
boiling fraction comprising acetic acid and anhydride, preferably
one comprising 0.5-5% acetic acid, and 90-100% of this higher
boiling fraction together with fresh acetic anhydride to give a
recycle stream comprising up to 5% acetic acid is recycled into the
reaction vessel.

The reaction is performed usually until at least 70% e.g. at least
80% or 90% conversion to the tetraacetylalkylenediamine has
occurred. Preferably it is carried out until analysis of a sample
of the reaction mixture shows that less than 5% of the original
amide is present as triacetyl alkylene diamine, preferably less than
3% e.g. less than 2%, and the amount of acetic acid is less than 3%
e.g. less than 2% by weight of the total of acetic acid and acetic
anhydride. Usually a reaction time of 6-15 hrs. or 6-14 hrs. and

7

**0079671**

especially 8-15, 8-12 hrs. or 8-10hrs. is used preferably with a reaction vessel temperature at 140-160$^\circ$C e.g. 140-150$^\circ$C. Though a volatile solvent capable of entraining acetic acid to aid its removal in the vapour may be used, the reaction is usually performed in the absence of said entrainer for acetic acid apart from acetic anhydride. Ketene is also usually absent. The acetic acid is evaporated as fast as possible with the aid of the acetic anhydride as entraining agent especially over the last 50% of reaction.

At the end of the reaction, the mixture may be cooled e.g. to 0-40$^\circ$C and crystals of tetracetyl alkylene diamine separated from the mother liquor. Preferably however, at the end, the mixture is heated to remove substantially all the remaining acetic acid (if any) and often concentrated e.g. to 40-50% by weight tetra acetyl alkylene diamine. The concentrated liquor is then cooled e.g. to - 0-40$^\circ$C and crystals of tetra acetyl alkylene diamine separate from the mother liquor. Yields of 93-97% of the crystals may be obtained with the mother liquor containing a further 1% of the tetra compound and coloured materials, but essentially no tri acetyl alkylene diamine. The mother liquor may be treated to recover a further crop of crystals of tetra compound, but because of the presence of the coloured materials, this treatment is preferably not employed. If desired the crystals may be recrystallized.

The process of the invention may be performed in an apparatus as shown schematically in the accompanying drawings in which a reaction vessel 1 has inlet lines 2 and 3 for acetic anhydride and diacetyl alkylene diamine, exit vapour line 4, entry liquid line 14 and exit line 17. Line 4 joins vessel 1 with a fractionating column 5 from the top of which comes a line 6 leading to a condenser 7, and thence via line 8 to a tank 9 and via a line 10 into a column 11, from which there is an overhead vapour line 12 and a liquid line 13 which leads into a tank 16, into which there is another entry line 15 and a recycle line 14 returning to vessel 1.

The apparatus is maintained under substantially anhydrous conditions by means not shown.

In use, acetic anhydride and diacetyl alkylene diamine are added into vessel 1 through lines 2 and 3 respectively. The mixture obtained is heated and the vapours of acetic acid and anhydride leave via line 4 and are crudely fractionated in column 5 to give an overhead vapour in line 6 substantially richer in acetic acid than was that in line 4. The vapour in line 6 is condensed in condenser 7, the condensate passed to tank 9 from which the condensate is passed via line 10 into fractionating column 11. Alternatively but less preferred (not shown) the vapour in line 6 may be passed itself directly into column 11. In column 11 there is separated an overhead vapour, which comprises acetic acid, which leaves via line 12, from a liquid leaving via line 13 from the base of column 11, said liquid comprising acetic anhydride and a little acetic acid. The liquid in line 13 may be completely recycled via tank 16 and line 14 to vessel 1 or may be mixed in tank 16 with fresh and/or distilled acetic anhydride added via line 15. At the end of the reaction the recycle flow through line 14 is stopped and all the liquid from column 11 is removed via line 3. The liquid in vessel 1 is concentrated to remove acetic acid and increase the concentration of amide therein. To encourage this removal of acetic acid, fresh acetic anhydride may be added via line 2 and the distillation continued to reduce further the amount of acetic acid in vessel 1.

Alternatively when the reaction is substantially complete, the recycle flow is reduced to lower the acetic acid content of the reaction mixture, e.g. to less than 2% and to increase the tetra acetyl alkylene diamine content, and then finally the recycle flow is stopped. The liquid in vessel 1 is then passed out of vessel 1 through line 17 and is allowed to cool or is cooled, by means not shown, with stirring of the liquid and the slurry of crystals and mother liquid is separated by means not shown to give crystals of tetra acetyl alkylene diamine.

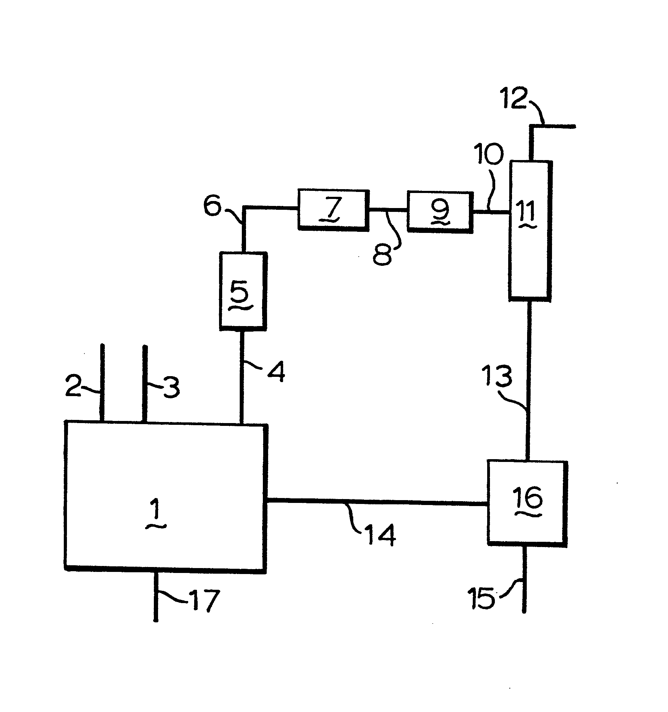

The invention is illustrated in the following Examples, in which reference in Example 1 is made to apparatus as illustrated in the accompanying drawing.

Example 1

Diacetyl ethylene diamine was made by heating under a nitrogen blanket ethylene diamine (1 mole) with acetic acid (2.25 mole) and water with distillation of water and acetic acid until no more water was evaporated at 125-180°C pot temperature. The reaction mixture was evaporated to dryness under vacuum to leave crude pale yellow diacetylethylene diamine of 97% purity with 1% water, 1% acetic acid and 1% 2-methyl imidazoline.

Crude diacetyl ethylene diamine (1 mole) was mixed with acetic anhydride (4 mole 98% pure 2% acetic acid) in vessel 1 and heated at about 145°C for 10 hrs under partial reflux. The vapours from the top of column 5 consisted of a mixture of acetic acid and anhydride and were condensed in condenser 14 and the condensate passed to tank 15. Periodically during the reaction the condensate in the tank was fractionated in column 7 into a 95 : 5 w/w mixture of acetic acid and anhydride as overhead vapour and a liquid underflow of 95 : 5 v/v mixture of acetic anhydride and acetic acid. The latter mixture is passed into tank 12, where it was mixed with make up acetic anhydride and a 98 : 1 w/w mixture of acetic anhydride and acetic acid was recycled to vessel 1 at a rate to maintain constant the liquid level in vessel 1. Over the course of the reaction a total of 2 moles of make up acetic anhydride was passed into vessel 1. Towards the end of the 10 hr. heating, the recycle flow in line 11 was adjusted so that at the end of the 10 hr reaction time the liquid in vessel 1 contained a solution of 45% tetra acetyl ethylene diamine 0.45% acetic acid and 0.5% tri acetyl ethylene diamine. The concentrated liquid was passed into crystallizer in which crystals of tetra acetyl ethylene diamine were deposited from the mother liquor were subsequently separated in 96% molar yield, and after they had been washed with acetic anhydride yielded white crystals of m.p. 149°C.

Example 2

Crude diacetylethylenediamine was prepared according to the method given in Example 1 and contained 2-methyl Imidazoline (1.3%), water (0.7%) and acetic acid (4.0%). 338.4g of the crude diacetylethylenediamine (2.21 mol) was mixed with 852.4g commercial acetic anhydride (8.19 mol 98% pure, 2% acetic acid) and the mixture heated at approximately 140-150°C for 10 hrs. in a reaction flask equipped with dropping funnel, thermometer, stirrer, and a short fractionating column with a partial take-off reflux head. A distillate consisting of a mixture of acetic anhydride and acetic acid was removed during the reaction at an approximate rate of 100g/hr. and the volume in the flask was maintained constant by the addition of fresh acetic anhydride, a total of 943.5g (9.05 mole) being added throughout the reaction.

At the end of the reaction the flask was cooled with stirring to 20°C when crystals of tetra acetylethylene diamine separated out. The slurry was filtered and the crude tetra acetylethylene diamine was washed with acetic anhydride and dried to yield tetra acetylethylene diamine (481.3g a yield of 95.5%).

Example 3

The procedure described in Example 2 was followed with the exception that a purer diacetyl ethylene diamine and a higher mole ratio of acetic anhydride: diacetylethylenediamine were used. The
diacetylethylenediamine contained 2-methyl Imidazoline (0.7%), water
(0.5%) and acetic acid (0.8%). The initial reaction mixture
consisted of 338.5g crude diacetylethylenediamine (2.3 mol) and
958.8g commercial acetic anhydride (9.21 mol 98% pure 2% acetic
acid). During the reaction 862g (8.3 mole) acetic anhydride was
added to maintain a constant volume in the flask to compensate for
the 100g/hr. distillate removed. At the end of the preparation
acetic anhydride was distilled off to yield an approximately 45%
solution of tetra acetylethylene diamine. After isolation there was
obtained tetra acetyl ethylene diamine (504.4g, a yield of 96%).

Example 4

The procedure described in Example 2 was followed with the
exceptions that a different purity diacetyl ethyelendiamine and a
higher mole ratio of acetic anhydride: diacetylethylenediamine were
used. The diacetylethylenediamine contained 2-methyl imidazoline
(1.3%), water (1.3%) and acetic acid (2.8%). The initial reaction
mixture consisted of 239.9g of crude diacetylethylenediamine (1.58
mol) and 959.5g commercial acetic anhydride (9.32 mol 99.1% pure
0.9% acetic acid). During the reaction 1242.5g (12 moles) acetic
anhydride was added to maintain the constant volume in the flask.
At the end of the preparation acetic anhydride was distilled off to
yield an approximately 45% solution of tetra acetylethylene diamine.
After isolation there was obtained tetra acetylethylenediamine
(351g, a yield of 97.5%).

Example 5

The progress with time of the reaction in Example 3 was followed and
the distillate volume and acetic acid content and the analysis of
the reaction mixture were monitored. The results were as follows.

0079671

| Time | Distillate | | Reactant Mixture % wt. | | | |
|------|------------|-------|------|--------|------|------|
| (Mins) | % of total AcOH removed | %AcOH | TAED | TriAED | DAED | AcOH |
| 30 | 5.9 | 36.2 | 9.2 | 18.2 | 5.0 | 13.0 |
| 60 | 18.3 | 50.4 | - | - | - | - |
| 90 | 28.2 | 59.7 | - | - | - | - |
| 114 | - | - | 20.3 | 12.1 | 1.4 | 11.8 |
| 174 | 56.3 | 58.1 | 25.7 | 8.7 | 0.7 | 8.0 |
| 234 | 73.6 | 47.0 | 31.8 | 6.3 | - | 4.6 |
| 294 | 82.8 | 37.8 | 35.0 | 4.1 | - | 2.9 |
| 354 | 89.0 | 24.4 | 35.9 | 2.9 | - | 2.1 |
| 429 | 93.7 | 17.6 | 37.4 | 2.0 | - | 1.2 |
| 549 | 98.3 | 9.3 | 38.5 | 1.0 | - | 0.8 |
| 599 | 100 | 4.7 | - | - | - | - |

TAED, TriAED, DAED and AcOH mean respectively tetra-, tri- and di-acetylethylene diamine and acetic acid respectively.

Claims

1.  A process for preparing a tetraacetyl alkylene diamine with 1-6 carbon atoms in the alkylene group, which process comprises heating a mixture comprising an amide which is a diacetylalkylenediamine with acetic anhydride to form a reaction mixture comprising at least one amide and acetic acid while evaporating a vapour comprising acetic acid and then recovering tetraacetylalkylenediamine from said reaction mixture characterized in that the diacetylalkylene diamine is heated with acetic anhydride in a molar ratio of 1 : 2 to 1 : 10 while evaporating a vapour comprising acetic acid and acetic anhydride and adding further acetic anhydride to said reaction mixture during the reaction and maintaining a molar ratio of acetic anhydride to the total of amides containing said alkylene group at 3 : 1 to 10 : 1 during the reaction.

2.  A process according to claim 1 characterised in that acetic acid and acetic anhydride are distilled off said reaction mixture.

3.  A process according to claim 1 or 2 characterised in that at least some of the acetic acid is separated from said vapour or a liquid condensate thereof.

4.  A process according to claim 3 characterised in that the vapour or liquid condensate thereof is separated into a lower boiling fraction comprising acetic acid and a higher boiling fraction comprising acetic anhydride.

5.  A process according to claim 4 characterised in that said higher boiling fraction comprising acetic anhydride is mixed with further acetic anhydride to form a stream, which is recycled into the reaction mixture.

6. A process according to claim 4 or 5 characterised in that the further acetic anhydride added to the reaction mixture contains 0.5-5% acetic acid (by weight).

7. A process according to any one of claims 1-6 characterised in that the reaction mixture is heated in apparatus with a condenser to cause partial reflux of the vapour therefrom and there is removed from said condenser a vapour overhead comprising acetic acid in higher percentage than said vapour from the reaction mixture.

8. A process according to any one of claims 1-6 characterised in that the reaction is performed in the absence of ketene or an entrainer, for acetic acid, apart from acetic anhydride.

9. A process according to any one of claims 1-7 characterised in that a molar ratio of acetic anhydride to the total amides containing said alkylene group of 3 : 1 to 5 : 1 is maintained during said reaction.

10. A process according to any one of claims 1-9 characterised in that said reaction is performed at 140-160°C for 8-15 hrs.

11. A process according to any one of claims 1-10 characterised in that the rate of removal of acetic acid is such that during at least 70% of the reaction, the rate of removal is greater than the rate of production of acetic acid.

12. A process according to any one of claims 1-11 wherein the rate of removal of acetic acid is such that after 80% of reaction the percentage of acetic acid is less than 5%.

13. A process according to any one of claims 1-12 characterised in that tetraacetyl ethylenediamine is prepared.

0079671